(19) Europäisches Patentamt
European Patent Office
Office européen des brevets

(11) EP 4 715 835 A1

(12) EUROPEAN PATENT APPLICATION

(43) Date of publication:
**25.03.2026 Bulletin 2026/13**

(21) Application number: **24201053.6**

(22) Date of filing: **18.09.2024**

(51) International Patent Classification (IPC):
***G16H 20/30*** *(2018.01)* ***G16H 50/30*** *(2018.01)*
***G06N 3/08*** *(2023.01)* ***G06N 20/00*** *(2019.01)*

(52) Cooperative Patent Classification (CPC):
**G16H 20/30; G06N 3/044; G06N 3/08; G06N 20/00; G16H 50/30**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Imec VZW**
  **3001 Leuven (BE)**
- **Universiteit Antwerpen**
  **2000 Antwerpen (BE)**

(72) Inventors:
- **VERDONCK, Tim**
  **3001 Leuven (BE)**
- **LUTIN, Erika**
  **3001 Leuven (BE)**
- **SERVOTTE, Thomas**
  **3001 Leuven (BE)**
- **NONNEMAN, Bart**
  **3001 Leuven (BE)**
- **COLLE, Camille**
  **3001 Leuven (BE)**
- **KHOLKINE, Leonid**
  **3001 Leuven (BE)**
- **LATRE, Steven**
  **3001 Leuven (BE)**

(74) Representative: **Ipsilon Belgium**
**Bellevue 5/501**
**9050 Gent-Ledeberg (BE)**

# (54) DETERMINING ENDURANCE PERFORMANCE BY A RECURRENT NEURAL NETWORK

(57) Example embodiments relate to a computer-implemented method for determining endurance performance of a subject based on sensor measurements; the computer-implemented method comprising: obtaining (201) a training dataset (214) comprising workload data (212) and heart rate data (211) measured, by respectively at least one workload sensor and at least one heart rate sensor, during respective physical activities performed by the subject within a historical window; training (202) a recurrent neural network, RNN (220), based on the training dataset (214); wherein the RNN (220) is configured to determine the heart rate (221) of the subject at an exerted workload (222) considering a historic time series of heart rate data (223) and workload data preceding the exerted workload; and determining (203) at least a portion of a workload-heart rate relationship (263), indicative for the endurance performance of the subject, by feeding one or more fixed workload time series (241, 242, 243) to the trained RNN (220).

Obtaining a training dataset
Training Recurrent Neural Network
Determining at least a portion of a workload-heart rate relationship
RNN
WORKLOAD
TIME
HEART RATE
WORKLOAD

Fig. 2



Processed by Luminess, 75001 PARIS (FR)

## Description

### Field of the Invention

The present invention generally relates to determining the endurance performance of a subject using sensor data, in particular to determining at least a portion of a workload-heart rate relationship of the subject based on heart rate data and workload data.

### Background of the Invention

The endurance performance of endurance athletes is, amongst others, characterized by an aerobic lactate threshold and an anaerobic lactate threshold, also referred to as LT1 and LT2 respectively. The aerobic lactate threshold expresses the minimum workload an endurance athlete has to exert to reach lactate production levels that exceed his/her physiological baseline levels. The anaerobic lactate threshold expresses the workload at which lactate production starts to exceed lactate clearance, thereby resulting in rapid lactate accumulation.

Training plans and structured workouts are typically constructed based on the lactate thresholds of the athlete, in particular on the workload an athlete can exert at the anaerobic threshold. However, accurately determining the lactate thresholds typically requires performing an exercise test at incremental workloads during which blood samples of the athlete are regularly collected. The lactate concentration within the blood samples can then be related to the incremental workloads. These exercise tests have the problem that they are costly, typically require the athlete to perform a maximal effort, have to be performed in a controlled environment, and are generally perceived as unpleasant by most athletes. As such, these tests are rarely performed by recreational athletes and even infrequently performed by professional athletes.

Some methods exist to approximate the anaerobic lactate threshold with a proxy value based on a dedicated test protocol without collecting blood samples. For example, in cycling, a common approach is to perform a ramp-up test where the workload is incrementally increased until failure. This allows determining the power a cyclist can hold for an hour, i.e. the functional threshold power, which can be used as an approximation of the workload at the anaerobic threshold. However, this has the problem that it requires the athlete to perform a maximal effort during a dedicated test, which is undesired as it results in undue fatigue of the athlete thereby interfering with optimal endurance training. This further makes it unsuitable for continuous performance tracking as the negative effects of frequently repeating such maximal efforts are even more undesirable.

Instead of a dedicated test protocol, some methods attempt to approximate the lactate thresholds of an athlete based on data obtained during regular workouts, e.g. by determining a power-duration curve or by analysing the heart rate variability. However, this has the problem that athletes typically do not spend a lot of time in high-intensity zones during regular workouts and therefore still require incorporating regular maximal efforts in their training plan to enable approximation of the anaerobic threshold. Additionally, these approximation methods are typically sensitive to outliers and noise.

It is a further problem that these approximation methods only allow approximating the anaerobic threshold in an individualized manner, and that the aerobic threshold is merely estimated in a non-individualized manner using population-based heuristics.

### Summary of the Invention

It is an object of the present invention, amongst others, to solve or alleviate above identified problems and challenges by determining the endurance performance of a subject based on sensor measurements obtained during regular workouts without requiring regular maximal efforts, regular high-intensity efforts, or invasive blood sampling.

According to a first aspect, this object is achieved by a computer-implemented method for determining endurance performance of a subject based on sensor measurements. The computer-implemented method comprising:

- obtaining a training dataset comprising workload data and heart rate data measured, by respectively at least one workload sensor and at least one heart rate sensor, during respective physical activities performed by the subject within a historical window;
- training a recurrent neural network, RNN, based on the training dataset; wherein the RNN is configured to determine the heart rate of the subject at an exerted workload considering a historic time series of heart rate data and workload data preceding the exerted workload; and
- determining at least a portion of a workload-heart rate relationship, indicative for the endurance performance of the subject, by feeding one or more fixed workload time series to the trained RNN.

The workload data expresses an external effort the subject exerts during the physical activity. The workload data may therefore depend on the performed endurance sport, e.g. power in cycling or rowing, and pace in running or swimming. The workload data may further account for other external effort metrics during an endurance sport, e.g. elevation may be considered in addition to power or pace. The workload data is obtained by at least one workload sensor such as, for example, crank-based power meters in cycling, pedal-based power meters in cycling, a power meter in rowing, or a smart watch configured to measure pace in running or swimming.

The heart rate data expresses an internal effort the subject exerts during the physical activity. In other words, the heart rate data is the cardiovascular response of the subject to the workload of the physical activity. Thus, the workload data and the heart rate data are time series with corresponding time steps. The heart rate data is obtained by at least one heart rate sensor, e.g. a pulse oximeter or photoplethysmography, PPG, sensor provided within a smart watch; or an electrocardiographic sensor provided within a chest strap. The at least one workload sensor and the at least one heart rate sensor may further be provided within a single device, e.g. a smart watch. It is an advantage that the computer-implemented method is agnostic to the used at least one heart rate sensor and the at least one workload sensor.

The workload data and heart rate data are measured during respective physical activities, also referred to as workouts. These physical activities have been performed by the subject within a historical time window. In other words, workload data and heart rate data measured during past workouts performed within the historical window are obtained to form the training dataset. The historical time window may preferably be large enough as to provide sufficient training data for accurately training the recurrent neural network, RNN. The historical window may, for example, be at least 25 weeks. A sufficient length of the historical window may further depend on the number of physical activities performed during that time window, e.g. a sufficient length of the historical window may be larger when the subject only performs 1 workout per week compared to a subject that performs 3 workouts per week.

The training dataset is then used for training the recurrent neural network. The trained RNN is configured to determine or predict the heart rate of the subject at a certain exerted workload, considering the heart rate and workload preceding that certain exerted workload. In other words, the trained RNN allows predicting the heart rate of the subject at a certain workload considering a recent history of workload and heart rate. In doing so, the RNN can account for cardiovascular drift and/or fatigue. The RNN may preferably be structured according to a long short-term memory, LSTM, architecture.

The trained RNN is then provided with one or more fixed workload time series as input as to determine at least a portion of the workload-heart rate relationship of the subject. A workload-heart rate relationship expresses the relationship between the workload exerted by a subject during a physical activity and the resulting heart rate of the subject. The workload-heart rate relationship is a good indicator of the endurance performance of the subject, as the workload-heart rate relationship can provide insight into the efficiency, endurance, fatigue resistance, training adaptations, and lactate thresholds of the subject.

The fixed workload time series simulate the subject holding a certain workload for a certain duration. In response to the fixed workload time series, the trained RNN thus outputs respective heart rate time series indicative for the predicted heart rate of the subject when holding the fixed workloads. The predicted heart rate time series converge gradually to respective steady-state values. The duration of the fixed workload time series may be sufficient to allow the predicted heart rates to converge to a steady-state value. This steady-state or converged heart rate value may then be related to the respective fixed workloads of the workload time series to obtain workload-heart rate pairs. In doing so, at least a portion of the workload-heart rate relationship can be determined as the workload-heart rate pairs represent data points of the workload-heart rate relationship. By feeding the RNN with a plurality of fixed workload time series at respective incremental workloads, a performance test may further be simulated without requiring the subject to actually perform a dedicated performance test such as, for example, the ramp-up test in cycling.

The computer-implemented method thus allows obtaining at least a portion of the workload-heart rate relationship indicative for the endurance performance of the subject without interfering with endurance training, as frequently performing maximal and/or high-intensity efforts is unnecessary. This has the further advantage that it makes the computer-implemented method suitable for continuous performance tracking. It is a further advantage that the performance of the subject can be determined and tracked based on regular physical activities performed in real-world environments, i.e. without structured training sessions or without performing tests in a controlled lab environment.

According to an example embodiment, obtaining the training dataset may comprise selecting respective physical activities performed within the historical window that meet a set of selection criteria.

According to an example embodiment, the set of selection criteria may comprise at least one of:

- a minimum duration of the respective physical activity;
- a maximum amount of missing and/or null workload datapoints within the workload data of the respective physical activity;
- a maximum amount of missing and/or null heart rate datapoints within the heart rate data of the respective physical activity;
- a heart rate range or a workload range;

- a maximum variation in the workload data of the respective physical activity;
- a minimum variation in the heart rate data of the respective physical activity; and
- a maximum altitude during the respective physical activity.

This allows improving the training data set by ignoring irrelevant heart rate data and workload data of respective physical activities that deteriorate the quality and accuracy of the trained recurrent neural network. As such, selecting physical activities according to the selection criteria can further improve the accuracy and reliability of the determined portion of the workload-heart rate relationship.

According to an example embodiment, obtaining the training dataset may comprise oversampling the workload data and the heart rate data of physical activities that exceed a predetermined intensity.

Oversampling the workload data and the heart rate data of a physical activity may, for example, be achieved by duplicating the workload data and the heart rate data of a high-intensity physical activity. The intensity of a physical activity may be determined by the amount of time spend above certain workloads and/or heart rates during the physical activity. The predetermined intensity may thus, for example, be a workload threshold or a heart rate threshold that is exceeded for a certain period of time during the physical activity.

This allows oversampling physical activities comprising efforts that are typically underrepresented in regular physical activities, e.g. high intensity intervals close to or above the anaerobic threshold. This has the advantage that the recurrent neural network may be trained on more high intensity training data without requiring the subject to perform a substantial amount of high intensity efforts.

According to an example embodiment, the oversampling may be performed on physical activities that have heart rate data values that exceed a threshold percentile value of all heart rate data values within the training dataset during at least a predetermined portion of the physical activity.

A physical activity may, for example, be oversampled if the heart rate data values during at least 20% of the duration of the physical activity exceed the $85^{th}$ percentile of all heart rate data values within the training dataset.

According to an example embodiment, obtaining the training dataset may comprise selecting respective initial portions of the workload data and the heart rate data of the respective physical activities; wherein the initial portions have a predetermined length.

The predetermined length may, for example, be one hour. By selecting initial portions of predetermined length, the workload data and heart rate data of the respective physical activities upon which the recurrent neural network is trained have the same length. This allows batched training of the recurrent neural network and reduces training time. Selecting an initial portion of the workload data and the heart rate data of the respective physical activities further avoids that delayed-onset physiological responses that negatively affect performance, e.g. muscle fatigue, lactate buildup, and dehydration, are included unevenly within the training dataset thereby avoiding that these processes distort the training of the recurrent neural network.

According to an example embodiment, obtaining the training dataset may comprise weighting the heart rate data values within the training dataset inversely according to their frequency.

In other words, heart rate values that occur less receive a higher weight, while heart rate values that occur often receive a lower weight. In doing so, more weight is assigned to rare heart rate zones that are typically underrepresented in regular physical activities, e.g. relatively high and relatively low heart rates. The lowest heart rate zone, e.g. below the $10^{th}$ percentile, may preferably be excluded from the weighting as these values are less relevant to determine the performance of the subject and often include heart rate sensor malfunctions. This may further improve the accuracy of the determined workload-heart rate relationship in the high intensity region, i.e. around the anaerobic threshold, without requiring the athlete to perform maximal efforts.

According to an example embodiment, the weighting may be performed on heart rate data values exceeding the $10^{th}$ percentile of all heart rate data values within the training dataset.

According to an example embodiment, training the RNN may further comprise finetuning one or more layers of the RNN on a subset of most recent physical activities within the training dataset.

This can be achieved by setting the weights of the one or more finetuned layers of the RNN to trainable while fixing the weights of the other layers of the RNN. The subset of most recent physical activities may, for example, comprise a predetermined number of most recent physical activities or the physical activities performed during a most recent period. The finetuning may, for example, be performed after logging the workload data and heart rate data of a new physical activity. Alternatively or complementary, the finetuning may be performed at predetermined intervals. This allows updating the trained RNN to the current performance level of the subject and, thus, allows updating the workload-heart rate relationship according to the current performance level of the subject.

According to an example embodiment, the computer-implemented method may further comprise detecting deviations within a most recent physical activity by excluding the most recent physical activity from the training dataset and comparing the heart rate data measured during the most recent physical activity with the heart rate data determined by the RNN when feeding the workload data measured during the most recent physical activity to it.

In other words, the heart rate measured during the most recent physical activity may be compared with the predicted heart rate by the RNN. Deviations may occur when the subject has overperformed or underperformed during the most recent physical activity, i.e. when the subject had a particularly good or bad workout. As such, the comparison between measured heart rate data and predicted heart rate data can allow to detect overperformance and underperformance. Alternatively, deviations may occur due to measurement errors such as a malfunctioning heart rate sensor or due to other environmental factors that impact the subject's performance such as weather conditions.

According to an example embodiment, the computer-implemented method may further comprise determining the workload of the subject at an aerobic threshold based on the determined workload-heart rate relationship and a predetermined heart rate of the subject at the aerobic threshold.

In other words, the heart rate of the subject at the aerobic threshold should be known. This predetermined heart rate may, for example, be obtained by performing a single lactate test in a lab. Thereafter, the predetermined heart rate at the aerobic threshold may be used to derive the workload at the aerobic threshold from the determined workload-heart rate relationship by the trained RNN. This allows determining the workload at the aerobic threshold without repeating a lactate test and without performing dedicated test protocols in controlled circumstances, i.e. based on regular physical activities. This has the advantage that the workload at the aerobic threshold can be determined reliably and in an individualized manner.

According to an example embodiment, the computer-implemented method may further comprise determining the workload of the subject at an anaerobic threshold based on the determined workload-heart rate relationship and a predetermined heart rate of the subject at the anaerobic threshold.

In other words, the heart rate of the subject at the anaerobic threshold should be known. This predetermined heart rate may, for example, be obtained by performing a single lactate test in a lab. Thereafter, the predetermined heart rate at the anaerobic threshold may be used to derive the workload at the anaerobic threshold from the determined workload-heart rate relationship by the trained RNN. This allows determining the workload at the anaerobic threshold without repeating a lactate test and without performing dedicated test protocols in controlled circumstances, i.e. based on regular physical activities. This has the advantage that the workload at the anaerobic threshold can be determined reliably without having to perform dedicated maximal efforts.

According to an example embodiment, the computer-implemented method may further comprise determining the workload and/or the heart rate of the subject at an aerobic threshold and/or at an anaerobic threshold by identifying respective deflection points of the determined workload-heart rate relationship.

The lactate thresholds are located around respective deflection points of the workload-heart rate relationship. Identifying the deflection points may be achieved by identifying local extrema of the second order derivative of the determined workload-heart rate relationship. This allows determining the workload at the aerobic threshold and at the anaerobic threshold without any prior knowledge on the heart rate and/or workload at the lactate thresholds, i.e. without performing a single lactate test. Additionally, this further allows determining the heart rate at the aerobic threshold and at the anaerobic threshold without performing a single lactate test.

According to an example embodiment, the computer-implemented method may comprise determining parameter values of a fitness-fatigue model by minimizing a difference between the workload at the anaerobic threshold estimated by the fitness-fatigue model and the determined workload at the anaerobic threshold.

A fitness-fatigue model is a conceptual framework that describes how an athlete's performance is influenced by two competing factors: fitness and fatigue. A fitness-fatigue model predicts how training, i.e. performing physical activities, affects endurance performance over time by balancing the positive effects of fitness gains with the negative effects of accumulated fatigue. A typical fitness-fatigue model predicts a performance metric based on a relationship defined by parameter values and training load. Training load refers to the amount of stress exerted on a subject during a single physical activity or workout.

A problem with fitness-fatigue models is that the parameter values are typically determined in a non-individualized manner using population-based heuristics or averages, as there is no continuous performance metric available to be used as a target to fit the parameter values. As such, fitness-fatigue models with an individualized fit are rare. Available individualized fitness-fatigue models are impractical as they require a long period of frequent field or lab testing to obtain target performance metrics. Moreover, this frequent testing typically requires the subject to perform dedicated maximal efforts which is undesirable.

By using the workload at the anaerobic threshold determined based on the workload-heart rate relationship obtained by the RNN, a continuous performance metric target can be obtained without performing frequent maximal efforts, long periods of structured training, frequent field or lab testing, or additional measuring protocols outside regular physical activities or training.

According to an example embodiment, minimizing the difference may be performed by a differential evolution algorithm.

This allows determining the parameter values of the fitness-fatigue model in a stable and reliable manner.

According to an example embodiment, the computer-implemented method may further comprise determining a training load of at least one physical activity based on the workload data and/or the heart rate data of the at least one physical activity, wherein the training load is indicative for stress exerted on the subject by the at least one physical activity; and wherein the fitness-fatigue model determines the performance of a subject based on the parameter values and the training load.

According to an example embodiment, the determined parameter values of the fitness-fatigue model may comprise:

- a first scaling factor indicative for an increase in performance due to the fitness effect of the physical activity;
- a second scaling factor indicative for a decrease in performance due to the fatigue effect of the physical activity;
- a first time factor indicative for a decay of the impact of training load on the increase in performance over time; and
- a second time factor indicative for a decay of the impact of training load on the decrease in performance over time.

According to an example embodiment, determining the parameter values of the fitness-fatigue model further comprises constraining the values of the first scaling factor and the second scaling factor between at least 0.01 and at most 3.00, constraining the value of the first time factor between at least 10 days and at most 60 days, and constraining the value of the second time factor between at least 1 day and at most 10 days.

According to an example embodiment, the second scaling factor may be at least equal to the first scaling factor; and a difference between the first time factor and the second time factor may be at least 10 days.

According to an example embodiment, a performance metric estimated by the fitness fatigue model is defined as

$$P_0 + k_1 \sum_{i=1}^{n-1} w_i e^{-(n-i)/\tau_1} - k_2 \sum_{i=1}^{n-1} w_i e^{-(n-i)/\tau_2}$$

or as

$$P_0 + k_1 \frac{\sum_{i=1}^{n-1} w_i e^{-(n-i)/\tau_1}}{\sum_{i=1}^{n-1} e^{-(n-i)/\tau_1}} - k_2 \frac{\sum_{i=1}^{n-1} w_i e^{-(n-i)/\tau_2}}{\sum_{i=1}^{n-1} e^{-(n-i)/\tau_2}}$$

, wherein $P_0$ is indicative for the model offset, $k_1$ is the first scaling factor, $k_2$ is the second scaling factor, $\tau_1$ is the first time factor, $\tau_2$ is the second time factor, $w_i$ is the training load of physical activity *i*, and n is a unit of time.

According to a second aspect, the object is achieved by a data processing system configured to perform the computer-implemented method according to the first aspect.

According to a third aspect, the object is achieved by a computer program comprising instructions which, when the computer program is executed by a computer, cause the computer to perform the computer-implemented method according to the first aspect.

According to a fourth aspect, the object is achieved by a computer-readable medium comprising instructions which, when executed by a computer, cause the computer to perform the computer-implemented method according to the first aspect.

## Brief Description of the Drawings

Fig. 1 shows an example of a lactate curve indicative for the relationship between the workload exerted by a subject and the blood lactate concentration of the subject during a physical activity;

Fig. 2 shows steps of a computer-implemented method for determining the performance of a subject without interfering with endurance training, according to example embodiments;

Fig. 3 shows further steps of the computer-implemented method for obtaining the training dataset and training the recurrent neural network, according to example embodiments;

Fig. 4 shows further steps of the computer-implemented method for determining the workload of a subject at the aerobic threshold and/or the anaerobic threshold, according to example embodiments;

Fig. 5 shows further steps of the computer-implemented method for determining the workload and/or heart rate of a subject at the aerobic threshold and/or the anaerobic threshold, according to example embodiments;

Fig. 6 shows steps of a computer-implemented method for obtaining an individualized fitness-fatigue model, according to example embodiments; and

Fig. 7 shows an example embodiment of a suitable computing system for performing steps according to example aspects of the invention.

## Detailed Description of Embodiment(s)

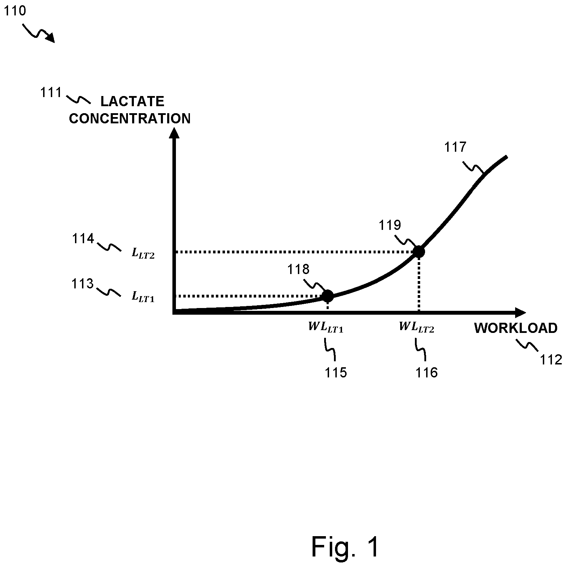

Fig. 1 shows an example of a lactate curve 117 indicative for the relationship between the workload 112 exerted by a subject and the blood lactate concentration 111 of the subject during a physical activity, e.g. cycling, running, swimming, rowing, or cross-country skiing. The lactate curve 117 comprises two points of particular interest for determining the endurance performance of an athlete: the aerobic lactate threshold 118 and the anaerobic lactate threshold 119. The aerobic lactate threshold 118 refers to the minimum workload an endurance athlete has to exert to reach blood lactate production levels that exceed his/her physiological baseline levels. The anaerobic lactate threshold 119 refers to the workload at which blood lactate production starts to exceed lactate clearance, thereby resulting in rapid lactate accumulation within the athlete. The anaerobic lactate threshold 118 typically occurs at a blood lactate concentration 113 of around 2.0 mmol/L, while the anaerobic lactate threshold 119 typically occurs at a blood lactate concentration 114 of around 4.0 mmol/L. However, this can vary between individuals.

The lactate thresholds 118, 119 are typically used to construct training plans and structured workouts for athletes. In particular, the workload 115, 116 an athlete can exert at the respective thresholds 118, 119 is typically used to plan structured training as it is a good indicator of the current endurance performance of the athlete. For example, in cycling, a structured training session may have the athlete perform 4 sets of 15 minute intervals between 70% - 85% of the workload 116 at the anaerobic threshold 119 with 5 minute recoveries between the intervals. As such, accurate and up-to-date knowledge of the lactate thresholds 118, 119 is important as it enables athletes to train optimally by correctly adjusting the intensity of their workouts to their current performance level. However, accurately determining the lactate thresholds 118, 119 typically requires performing an exercise test at incremental workloads during which blood samples of the athlete are regularly collected. This then allows determining the workload 112 the athlete can sustain at certain blood lactate levels 111, thereby obtaining the lactate curve 117

These exercise tests have the problem that they are costly, require the athlete to perform a maximal effort, have to be performed in a controlled environment, and are generally perceived as unpleasant by most athletes. Generally, it is undesirable to perform frequent maximal efforts, as these result in undue accumulation of fatigue and prevent the athlete to train optimally around the moment when the maximal effort has to be performed. In other words, the maximal effort of the exercise test to determine the anaerobic lactate threshold 119 interferes with optimal endurance training. This further makes the blood lactate exercise test unsuitable for continuous performance tracking, as the negative effects of frequently repeating such maximal efforts are even more undesirable. As such, these tests are rarely performed by recreational athletes and even infrequently performed by professional athletes.

Alternatives include approximating the lactate thresholds with a proxy value without collecting blood samples based on dedicated test protocols, e.g. the ramp-up test in cycling, or based on data obtained during regular workouts, e.g. by determining a workload-duration curve or by analysing the heart rate variability.

The approximation methods based on dedicated test protocols have the same problem as the blood lactate test in that they still require a maximal effort during a dedicated test, which interferes with optimal endurance training. The approximation methods based on regular training data have the problem that athletes typically do not spend a lot of time in high-intensity zones, i.e. around the anaerobic threshold 119, during regular workouts and therefore still require incorporating regular maximal efforts in their training plan to enable approximation of the anaerobic threshold 119. Additionally, these approximation methods are typically sensitive to outliers and noise.

It is a further problem that these approximation methods only allow approximating the anaerobic threshold in an individualized manner, and that the aerobic threshold is merely estimated in a non-individualized manner using population-based heuristics.

It can thus be desirable to determine and monitor the performance of a subject without interfering with optimal endurance training, i.e. without performing maximal efforts or high-intensity efforts solely for the purpose of determining the subject's performance.

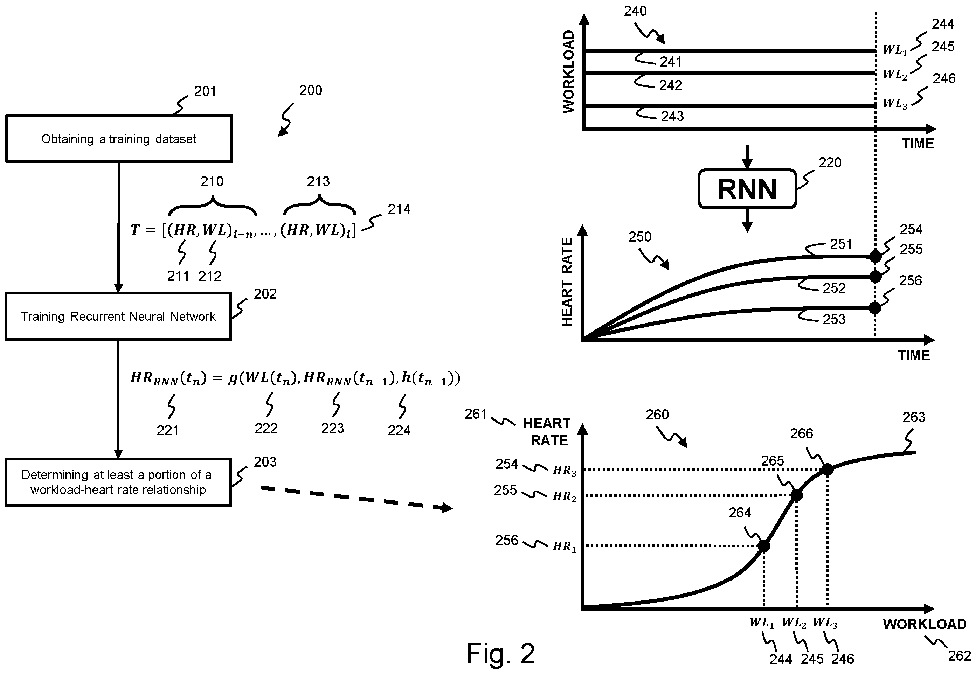

Fig. 2 shows steps 200 of a computer-implemented method for determining the endurance performance of a subject without interfering with endurance training, according to example embodiments.

In a first step 201, a training dataset 214 is obtained. The training dataset 214 comprises pairs 210, 213 of workload data 212 and heart rate data 211 measured during respective physical activities. The physical activities have been performed by the subject within a historical window. The historical window may have a certain length that defines the extend of past physical activities that are included within the training dataset 214. The historical window may, for example, be at least 25 weeks. As such, the training dataset 214 includes the workload and heart rate data of the physical activities performed in the past 25 weeks.

The workload data 212 expresses an external effort the subject exerts during the physical activity. The workload data 212 that is measured may therefore depend on the performed endurance sport, e.g. power in cycling or rowing, and pace in running or swimming. The workload data may further account for other external effort metrics during an endurance sport, e.g. elevation may be considered in addition to the pace or power. The workload data is obtained by at least one workload sensor. In cycling, power can for example be measured with a crank-based power meter or a pedal-based power meter. In running or swimming, pace can for example be measured with a smart watch.

The heart rate data 211 expresses an internal effort the subject exerts during the physical activity. The heart rate data thus represents the internal effort of the athlete to exert the external effort of the physical activity. In other words, the heart rate data is the cardiovascular response of the subject to the workload of the physical activity. It will thus be apparent that the workload data 212 and the heart rate data 211 are time series with corresponding time steps, i.e. the data points in the workload data 212 and heart rate data 211 are aligned in time such that each data point in the workload data 212 corresponds with a specific data point in the heart rate data 211. The heart rate data 211 is obtained by at least one heart rate sensor, e.g. a pulse oximeter or photoplethysmography, PPG, sensor provided within a smart watch; or an electrocardiographic sensor provided within a chest strap. The at least one workload sensor and the at least one heart rate sensor may further be provided within a single device, e.g. a smart watch. It is an advantage that the computer-implemented method is agnostic to the at least one heart rate sensor and the at least one workload sensor used.

In a next step 202, a recurrent neural network, RNN 220, is trained based on the training dataset 214. The RNN 220 is trained to determine the heart rate of the subject at an exerted workload considering a historic time series of heart rate data and workload data preceding the exerted workload. In other words, the trained RNN 220 allows determining or predicting the heart rate of the subject when exerting a certain workload at timestep $t$, while taking account of the workload and corresponding heart rate preceding timestep $t$. Considering such a historic time series of heart rate data and workload data preceding the exerted workload allows accounting for cardiovascular drift and/or fatigue. The RNN may preferably be structured according to a long short-term memory, LSTM, architecture. Preferably, the LSTM comprises four cells. The RNN may be trained using a gradient descent optimizer to minimize the mean square error of the predicted heart rate relative to the measured heart rate. Formally, the RNN may represent a function $g$ that predicts the heart rate $HR_{RNN}(t_n)$ 221 at timestep $t_n$ considering a history of workload and heart rate, i.e. $HR_{RNN}(t_n) = g(WL(t_n), HR_{RNN}(t_{n-1}), h(t_{n-1}))$ wherein $WL(t_n)$ 222 is the workload at timestep $t_n$, $HR_{RNN}(t_{n-1})$ 223 is the predicted heart rate at the preceding timestep $t_{n-1}$, and $h(t_{n-1})$ 224 is a hidden state of the RNN at the preceding timestep $t_{n-1}$ indicative for the history of workload and heart rate data. At each time step, the RNN updates the hidden state. In other words, the hidden state acts as a dynamic summary of the preceding workload data and heart rate data, thereby allowing to capture and propagate temporal dependencies such as cardiovascular drift and fatigue.

In a following step 203, at least a portion of the subject's workload-heart rate relationship 263 is determined. A workload-heart rate relationship expresses the relationship between the workload 262 exerted by a subject during a physical activity and the resulting heart rate 261 of the subject. The workload-heart rate relationship 263 is a good indicator of the endurance performance of the subject, as the workload-heart rate relationship can provide insight into the efficiency, endurance, fatigue resistance, training adaptations, and lactate thresholds of the subject.

Determining at least a portion of the subject's workload-heart rate relationship 263 is achieved by feeding one or more fixed workload time series 241, 242, 243 to the trained RNN 220. Fig. 2 illustrates an example wherein three fixed workload time series 241, 242, 243 are fed to the trained RNN 220. However, it will be apparent that more or fewer fixed workload time series can be fed to the trained RNN 220. The fixed workload time series 241, 242, 243 simulate the subject holding workload 244, 245, 246 for a certain duration. For each of the respective fixed workload time series 241, 242, 243, the trained RNN 220 predicts the heart rate 251, 252, 253 of the subject. In other words, the trained RNN outputs a heart rate time series 251, 252, 253 indicative for the predicted heart rate of the subject when holding the respective fixed workloads 241, 242, 243. For example, the trained RNN 220 predicts that the heart rate of the subject will follow curve 251 when he/she exerts workload 241.

The predicted heart rate time series 251, 252, 253 converge gradually to a substantially steady value $HR_3$ 254, $HR_2$ 255, and $HR_1$ 256, respectively. This is indicative for a physiological response delay during which the heart rate rises gradually to meet the increase demand for oxygen, also referred to as cardiac lag or heart rate lag. The duration or length of the fixed workload time series 241, 242, 243 may be sufficient to allow the predicted heart rates 251, 252, 253 to converge, i.e. to reach a steady state after the physiological response delay. These steady-state or converged heart rate values 254, 255, 256 may then be related to the fixed workloads $WL_1$ 244, $WL_2$ 245, $WL_3$ 246 of the respective workload time series 241, 242, 243, thereby forming workload-heart rate pairs $(WL_1,HR_1)$, $(WL_2,HR_2)$, and $(WL_3,HR_3)$. These respective workload-heart rate pairs each represent a data point 264, 265, 266 of the workload-heart rate relationship 263. As such, this allows determining at least a portion of the workload-heart rate relationship 263.

It will be apparent that the portion of the workload-heart rate relationship that is determined depends on the number of distinct fixed workload time series 241, 242, 243, the magnitude of the distinct workloads 244, 245, 246, and the range of the workloads 244, 245, 246 that are fed to the trained RNN 220. Feeding a plurality of fixed workload time series at distinct workloads can allow determining a substantial portion of the workload-heart rate relationship 263. Determining a portion of the workload-heart rate relationship may further include interpolating between the determined workload-heart rate pairs 264, 265, 266. Feeding the RNN 220 with a plurality of fixed workload time series at incremental workloads may simulate a dedicated performance test without requiring the subject to actually perform a dedicated performance test such as, for example, the ramp-up test in cycling.

In doing so, the computer-implemented method allows determining the endurance performance of a subject without interfering with optimal endurance training, i.e. without performing maximal efforts or high-intensity efforts solely for the purpose of determining the subject's performance. This has the further advantage that it makes the computer-implemented method suitable for continuous performance tracking, as frequent maximal or high-intensity efforts are not required. It is a further advantage that the performance of the subject can be determined and tracked based on regular physical activities performed in real-world environments, i.e. without structured training sessions or without performing tests in a controlled lab environment.

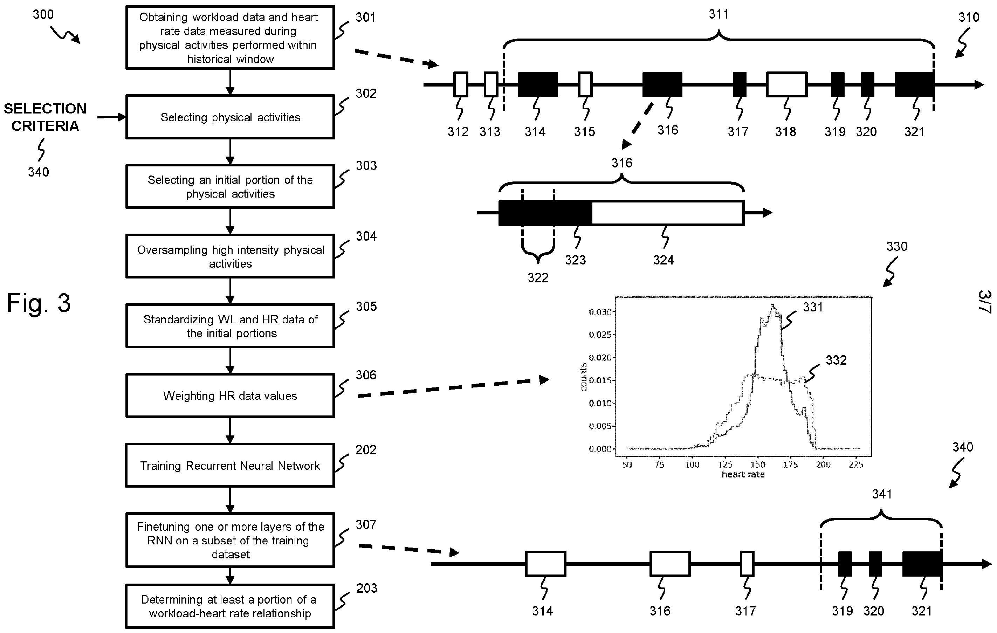

Fig. 3 shows further steps 300 of the computer-implemented method according to example embodiments. In particular, Fig. 3 shows further steps 301 - 306 for obtaining the training dataset, i.e. step 201 in Fig. 2. In a first step 301, workload data and heart rate data measured during physical activities 314 - 321 performed within the historical window 311 may be obtained. Earlier performed physical activities 312, 313 falling outside the historical window 311 may be ignored and are thus not included in the training dataset. In a next step 302, a subset of physical activities 314, 316, 317, 319, 320, 321 may be selected from the physical activities 314 - 321 performed within the historical window 311. The subset of physical activities 314, 316, 317, 319, 320, 321 may be selected based on a set of selection criteria 340.

A selection criterium may be a minimum duration of the respective physical activity, e.g. physical activities must be at least 15 minutes long. Another selection criterium may be a maximum amount of missing and/or null workload datapoints within the workload data of the respective physical activity, e.g. at most 30% missing and/or null values. Another selection criterium may be a maximum amount of missing and/or null heart rate datapoints within the heart rate data of the respective physical activity, e.g. at most 30% missing and/or null values. Another criterium may be a heart rate range or a workload range, e.g. physical activities with heart rate values below 40 bpm or above 220 bpm may be ignored as they are probably due to a sensor fault or malfunction. Another criterium may be a maximum variation in the workload data of the respective physical activity, e.g. at most a median second-to-second power difference of 50W. Another criterium may be a minimum variation in the heart rate data of the respective physical activity, e.g. less than 85% of 0 bpm second-to-second changes in heart rate. Another criterium may be a maximum altitude during the respective physical activity, e.g. at most 1000 m above sea level.

Selecting the physical activities 314, 316, 317, 319, 320, 321 based on the set of selection criteria 340 allows improving the training dataset by ignoring irrelevant heart rate data and workload data of respective physical activities 315, 318 that would deteriorate the quality and accuracy of the trained recurrent neural network. As such, this can improve the accuracy and reliability of the determined portion of the workload-heart rate relationship and, thus, improve the accuracy and reliability of determining the endurance performance of the subject.

In a next step 303, respective initial portions 323 of the physical activities 314, 316, 317, 319, 320, 321 may be selected. In other words, the workload data and the heart rate data of the selected physical activities 314, 316, 317, 319, 320, 321 that are included in the training dataset is limited to an initial portion 323. This avoids that delayed-onset physiological responses that negatively affect performance, e.g. muscle fatigue, lactate buildup, and dehydration, are included unevenly within the training dataset thereby avoiding distortion of the recurrent neural network training. The initial portions 323 may further have a predetermined length, e.g. one hour. In doing so, the workload data and heart rate data of the respective physical activities 314, 316, 317, 319, 320, 321 upon which the recurrent neural network is trained have the same length. This allows batched training of the recurrent neural network and reduces training time. Any missing values within the selected initial portions may further be imputed by interpolation. A moving average smoothing may further be applied to the selected initial portions of workload data, e.g. a 5s moving average smoothing.

In a next step 304, the workload data and the heart rate data of physical activities that exceed a predetermined intensity may be oversampled. Oversampling the workload data and the heart rate data of a physical activity may, for example, be achieved by duplicating the workload data and the heart rate data of a high-intensity physical activity. The intensity of a physical activity may be determined by the amount of time spend above certain workloads and/or heart rates during the physical activity. The predetermined intensity may thus, for example, be a workload threshold or a heart rate threshold that is exceeded for a certain period of time during the physical activity. In other words, a physical activity may, for example, be oversampled if the heart rate data values during at least a predetermined portion 322 of the physical activity 316 exceed a threshold percentile value of all heart rate data values within the training dataset, e.g. heart rate data exceeding the 85$^{th}$ percentile of all heart rate data values for at least 20% of the duration of the physical activity. It will be apparent that, if step 303 is performed, the condition for oversampling a physical activity applies only to the selected initial portion 323 of the physical activity 316. In other words, a portion 322 of the initial portion 323 should exceed the predetermined intensity.

This allows oversampling physical activities comprising efforts that are typically underrepresented in regular physical activities, e.g. high intensity intervals close to or above the anaerobic threshold. This has the advantage that the recurrent neural network may be trained on more high intensity training data without requiring the subject to perform a substantial amount of high intensity efforts. This may further improve the accuracy of the determined workload-heart rate relationship in the high intensity region, i.e. around the anaerobic threshold, without requiring the athlete to perform maximal efforts.

In a following step 305, the workload data and/or heart rate data of the initial portions 323 of the respective physical activities 314, 316, 317, 319, 320, 321 may be standardized. This can be achieved by subtracting the median and dividing by the interquartile range, i.e. by performing a robust standardization.

In a next step 306, the heart rate data values may be weighted inversely according to their frequency, i.e. the number of times a heart rate value occurs in the selected heart rate data. In other words, heart rate values that occur less receive a higher weight, while heart rate values that occur often receive a lower weight. This is illustrated in plot 330 of Fig. 3 which shows the original frequency 331 of the heart rate values and the weighted frequency 332 of the heart rate values. This allows assigning more weight to rare heart rate zones that are typically underrepresented in regular physical activities, e.g. relatively high and relatively low heart rate values.

The weighting may, for example, be performed on heart rate data values exceeding the $10^{th}$ percentile of all heart rate data values within the training dataset. The lowest heart rate zone, e.g. below the $10^{th}$ percentile, may preferably be excluded from the weighting as these values are less relevant to determine the performance of the subject and often include heart rate sensor malfunctions. This may further improve the accuracy of the determined workload-heart rate relationship in the high intensity region, i.e. around the anaerobic threshold, without requiring the athlete to perform maximal efforts.

In a following step 202, the RNN may be trained as described in relation to Fig. 2 on the training dataset obtained by performing steps 301 - 306. One or more layers of the trained RNN may further be finetuned in step 307. The finetuning may be performed based on a subset 341 of most recent physical activities 319, 320, 321 within the training dataset 340. The subset of most recent physical activities may comprise a predetermined number of most recent physical activities, e.g. the last ten physical activities of the subject. Alternatively, the subset of most recent physical activities may comprise the physical activities performed by the subject during a most recent period, e.g. during the last 4 weeks.

Finetuning the RNN can be achieved by setting the weights of the one or more finetuned layers of the RNN to trainable while fixing the weights of the other layers of the RNN. The finetuning may, for example, be performed after logging the workload data and heart rate data of a new physical activity. Alternatively or complementary, the finetuning may be performed at predetermined intervals. This allows updating the trained RNN to the current performance level of the subject and, thus, allows updating the workload-heart rate relationship according to the current performance level of the subject.

The computer-implemented method may further comprise detecting deviations within a most recent physical activity by comparing the heart rate data measured during a most recent physical activity with the heart rate data predicted by the RNN when feeding the workload data measured during the most recent physical activity to the RNN. In other words, the heart rate measured during the most recent physical activity may be compared with the predicted heart rate by the RNN. This most recent physical activity refers to the latest physical activity performed by the subject upon which the RNN has not been trained or updated yet. For example, detecting deviations may be performed for a physical activity (not shown in Fig. 3) following physical activity 321 using the RNN trained on the physical activities 314, 316, 317, 319, 320, 321.

Deviations may occur when the subject has overperformed or underperformed during the most recent physical activity, i.e. when the subject had a particularly good or bad workout. As such, the comparison between measured heart rate data and predicted heart rate data can allow to detect overperformance and underperformance. Alternatively, deviations may occur due to measurement errors such as a malfunctioning heart rate sensor or due to other environmental factors that impact the subject's performance such as weather conditions.

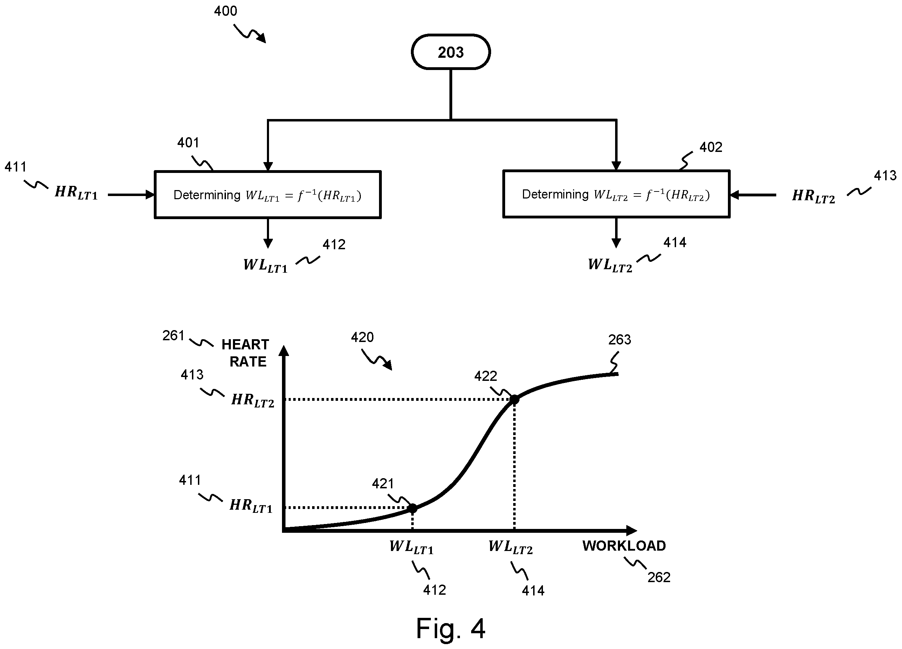

Fig. 4 shows further steps 400 of the computer-implemented method for determining the workload 412, 414 of the subject at the aerobic threshold 421 and/or the anaerobic threshold 422 based on the workload-heart rate relationship 263 $HR_{WL} = f(WL)$ determined in step 203. This can be achieved by determining the inverse of the workload-heart rate relationship 263, i.e. $f^{-1}(HR)$. This inverse relationship can then be used to determine the workload 412 of the subject at the aerobic threshold 421 in step 401 based on a predetermined heart rate 411 of the subject at the aerobic threshold 421. In other words, the heart rate of the subject at the aerobic threshold should be known.

This predetermined heart rate 411 may, for example, be obtained by performing a single lactate test in a lab. Thereafter, the predetermined heart rate at the aerobic threshold 411 may be used to derive the workload 412 at the aerobic threshold 421 from the workload-heart rate relationship 263 obtained by the trained RNN. This allows determining the workload 412 at the aerobic threshold 421 without repeating a lactate test and without performing dedicated test protocols in controlled circumstances, i.e. based on regular physical activities. This has the further advantage that the workload at the aerobic threshold can be determined reliably and in an individualized manner.

Alternatively or complementary, the inverse workload-heart rate relationship can be used to determine the workload 414 of the subject at the anaerobic threshold 422 in step 402, based on a predetermined heart rate 413 of the subject at the anaerobic threshold 422. In other words, the heart rate of the subject at the anaerobic threshold should be known. This predetermined heart rate 413 may, for example, be obtained by performing a single lactate test in a lab. Thereafter, the predetermined heart rate at the anaerobic threshold may be used to derive the workload 414 at the anaerobic threshold from the determined workload-heart rate relationship by the trained RNN. This allows determining the workload 414 at the anaerobic threshold 422 without repeating a lactate test and without performing dedicated test protocols in controlled circumstances, i.e. based on regular physical activities. This has the advantage that the workload at the anaerobic threshold can be determined reliably without having to perform maximal efforts.

It will be apparent that, for determining the workload 412, 414 at the aerobic threshold 421 or anaerobic threshold 422 based on the determined workload-heart rate relationship 263, a portion of the latter should at least be determined in step 203 around the aerobic threshold 421 or anaerobic threshold 422, respectively.

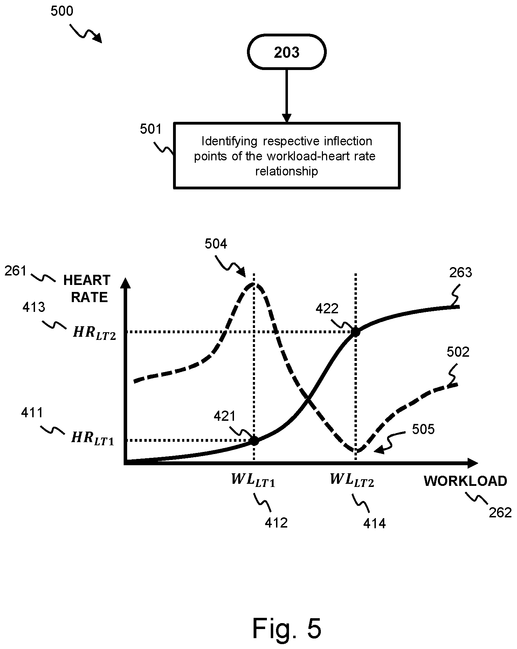

Fig. 5 shows alternative steps 500 of the computer-implemented method for determining the workload 412, 414 of the subject at the aerobic threshold 421 and/or the anaerobic threshold 422 based on the workload-heart rate relationship 263 determined in step 203.

To this end, deflection points of the workload-heart rate relationship 263 may be determined in step 501. A deflection point refers to a point on a curve where the direction of the curve changes, i.e. where there is a noticeable shift in the rate of change of the curve. A deflection point can thus be an inflection point or a point of substantial curvature change. The aerobic threshold 421 is typically located where the heart rate of the subject starts increasing more rapidly with increasing workload, i.e. where the slope of the workload-heart rate 263 increases. At workloads between the aerobic threshold 421 and anaerobic threshold 422 the workload-heart rate curve 263 is approximately linear. The anaerobic threshold 422 is typically located where the linear portion of the curve 263 ends and the slope decreases. The lactate thresholds are thus located at deflection points of curve 263 with substantial curvature changes.

Identifying the deflection points with substantial curvature changes in the workload-hear rate curve 263 may be achieved by identifying local extrema 504, 505 in the second order derivative 502 of the determined workload-heart rate relationship 263. A local maximum 504 in the second derivative 502 may be indicative for a maximum increase in the curvature of the workload-heart rate 263 curve, i.e. a substantial slope increase. A local minimum 505 in the second derivative 502 may be indicative for a maximum decrease in the curvature of the workload-heart rate 263 curve, i.e. a substantial slope decrease. Identifying these local extrema 504, 505 can thus allow determining the workload 412, 414 at the aerobic threshold 421 and at the anaerobic threshold 422 without any prior knowledge on the heart rate 411, 413 at the lactate thresholds, i.e. without performing a single lactate test. Additionally, this further allows determining the heart rate 411, 413 at the aerobic threshold 421 and at the anaerobic threshold 422 without any prior knowledge on the workload 412, 414 at the lactate thresholds, i.e. without performing a single lactate test.

The computer-implemented method thus allows continuously tracking the endurance performance of a subject by periodically determining a performance metric, e.g. the workload at the anaerobic threshold, without interfering with endurance training as frequently performing maximal and/or high-intensity efforts is unnecessary. The determined workload at the anaerobic threshold can be used as a continuous performance metric target for fitting an individualized fitness-fatigue model.

A fitness-fatigue model is a conceptual framework that describes how an athlete's performance is influenced by two competing factors: fitness and fatigue. A fitness-fatigue model predicts how training, i.e. performing physical activities, affects endurance performance over time by balancing the positive effects of fitness gains with the negative effects of accumulated fatigue. A typical fitness-fatigue model predicts a performance metric, e.g. the workload at the anaerobic threshold, based on a relationship defined by parameter values and training load. Training load refers to the amount of stress exerted on a subject during a single physical activity or workout.

A problem with fitness-fatigue models is that the parameter values are typically determined in a non-individualized manner using population-based heuristics or averages, as there are no methods to continuously track a performance metric to be used as a target to fit the parameter values without unduly interfering with the training of the subject. As such, fitness-fatigue models with an individualized fit are rare. Available individualized fitness-fatigue models are impractical as they require a long period of frequent field or lab testing to obtain a target performance metric. Moreover, this frequent testing typically requires the subject to perform a maximal effort which is undesirable.

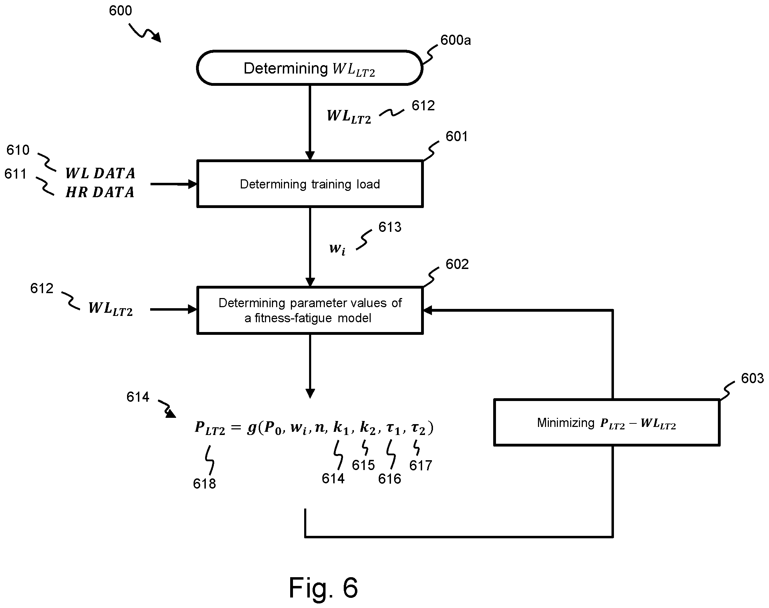

Fig. 6 shows steps 600 of a computer-implemented method for obtaining an individualized fitness-fatigue model according to example embodiments. To this end, the workload at the anaerobic threshold determined as described in relation to Fig. 4 or Fig. 5 may be obtained in step 600a. In a next step 601, the training load $w_i$ 613 of at least one physical activity may be determined based on workload data 610 and heart rate data 611 measured during the at least one physical activity. Preferably, the training load 613 is determined based on internal load techniques that capture the psychophysiological impact of the workload through subjective scores and heart rate, e.g. Banister's training impulse, bTRIMP, score. However, the training load 613 may also be determined based on external load techniques that quantify the objective workload, e.g. training stress score, TSS.

In the following steps 602 the parameter values of a fitness-fatigue model 614 may be determined. The fitness-fatigue model may be an equation that predicts the workload of a subject at the anaerobic threshold $P_{LT2}$ based on an initial base performance $P_0$, the training load $w_i$ of one or more physical activities $i$, a unit of time n, and parameter values 614 - 617. The parameter values 614 - 617 may preferably comprise a first scaling factor $k_1$ 614 indicative for an increase in performance due to the fitness effect of the physical activity; a second scaling factor $k_2$ 615 indicative for a decrease in performance due to the fatigue effect of the physical activity; a first time factor $\tau_1$ 616 indicative for a decay of the impact of training load on the increase in performance over time; and a second time factor $\tau_2$ 617 indicative for a decay of the impact of training load on the decrease in performance over time. The used fitness-fatigue model may be defined as

$$P_{LT2} = P_0 + k_1 \sum_{i=1}^{n-1} w_i e^{-(n-i)/\tau_1} - k_2 \sum_{i=1}^{n-1} w_i e^{-(n-i)/\tau_2} \qquad \text{(Eq. 1)}$$

Alternatively, the used fitness-fatigue model may be defined as

$$P_{LT2} = P_0 + k_1 \frac{\sum_{i=1}^{n-1} w_i e^{-(n-i)/\tau_1}}{\sum_{i=1}^{n-1} e^{-(n-i)/\tau_1}} - k_2 \frac{\sum_{i=1}^{n-1} w_i e^{-(n-i)/\tau_2}}{\sum_{i=1}^{n-1} e^{-(n-i)/\tau_2}} \qquad \text{(Eq. 2)}$$

The parameter values of the fitness-fatigue model may then be determined by minimizing 603 the difference between the workload at the anaerobic threshold estimated by the fitness-fatigue model $P_{LT2}$ and the determined workload 612 at the anaerobic threshold $WL_{LT2}$. In other words, the parameter values 614 - 617 may be iteratively adjusted as to minimize the difference between $P_{LT2}$ and $WL_{LT2}$. In doing so, an individualized fitness-fatigue model can be obtained without performing maximal efforts, long periods of structured training, frequent field or lab testing, or additional measuring protocols outside regular physical activities or training.

It will be apparent that steps 600 may be performed regularly as to continuously update the determined workload at the anaerobic threshold $WL_{LT2}$ and the parameter values 614 - 617 of the fitness-fatigue model. This has the advantage that personalized, and properly scaled fitness and fatigue variables can be determined and provided to athletes or coaches. This can offer useful applications in endurance training such as, for example, tracking the fitness/fatigue ratio as an indicator of overtraining.

Minimizing the difference in step 603 may further be performed by a differential evolution algorithm. This allows determining the parameter values 614 - 617 in a stable and reliable manner. Minimizing the difference in step 603 may further be subject to constraints based on physiological domain knowledge to limit the search space for the parameter values 614 - 617, thereby improving the convergence speed. The values of the first scaling factor $k_1$ 614 and the second scaling factor $k_2$ 615 may be constrained between at least 0.01 and at most 3.00, i.e. $k_1$ and $k_2 \in [0.01; 3.00]$. The values for the first time factor $\tau_1$ 616 may be constrained between at least 10 days and at most 60 days, i.e. $\tau_1 \in [10,60]$. The values for the second time factor $\tau_2$ 617 may be constrained between at least 1 day and at most 10 days, i.e. $\tau_2 \in [1, 10]$.

To further improve the identified parameter values 614 - 617 for the individualized fitness-fatigue model, minimizing the difference in step 603 may be subject to further constraints that only allow physiologically relevant combinations of the parameter values 614 - 617. These constraints may include that the second scaling factor $k_2$ 615 may be at least equal to the first scaling factor $k_1$ 614, i.e. $k_2 \geq k_1$; and a difference between the first time factor $\tau_1$ 616 and the second time factor $\tau_2$ 617 may be at least 10 days, i.e. $\tau_1 - \tau_2 \geq 10$.

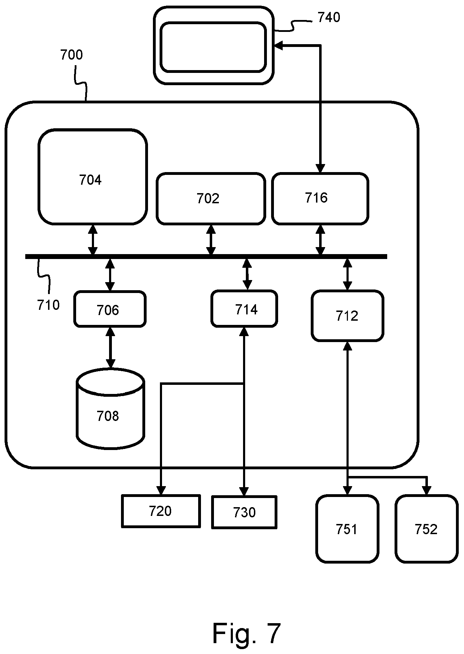

Fig. 7 shows a suitable computing system 700 enabling to implement embodiments of the above described computer-implemented method according to the invention. Computing system 700 may in general be formed as a suitable general-purpose computer and comprise a bus 710, a processor 702, a local memory 704, one or more optional input interfaces 714, one or more optional output interfaces 716, a communication interface 712, a storage element interface 706, and one or more storage elements 708. Bus 710 may comprise one or more conductors that permit communication among the components of the computing system 700. Processor 702 may include any type of conventional processor or microprocessor that interprets and executes programming instructions. Local memory 704 may include a random-access memory (RAM) or another type of dynamic storage device that stores information and instructions for execution by processor 702 and/or a read only memory (ROM) or another type of static storage device that stores static information and instructions for use by processor 702. Input interface 714 may comprise one or more conventional mechanisms that permit an operator or user to input information to the computing device 700, such as a keyboard 720, a mouse 730, a pen, voice recognition and/or biometric mechanisms, a camera, etc. Output interface 716 may comprise one or more conventional mechanisms that output information to the operator or user, such as a display 740, etc. Communication interface 712 may comprise any transceiver-like mechanism such as for example one or more Ethernet interfaces that enables computing system 700 to communicate with other devices and/or systems such as for example, amongst others, at least one heart rate sensor 751 and at least one workload sensor 752. The communication interface 712 of computing system 700 may be connected to such another computing system by means of a local area network (LAN) or a wide area network (WAN) such as for example the internet. Storage element interface 606 may comprise a storage interface such as for example a Serial Advanced Technology Attachment (SATA) interface or a Small Computer System Interface (SCSI) for connecting bus 710 to one or more storage elements 708, such as one or more local disks, for example SATA disk drives, and control the reading and writing of data to and/or from these storage elements 708. Although the storage element(s) 708 above is/are described as a local disk, in general any other suitable computer-readable media such as a removable magnetic disk, optical storage media such as a CD or DVD, -ROM disk, solid state drives, flash memory cards, etc. could be used.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

**1.** A computer-implemented method for determining endurance performance of a subject based on sensor measurements; the computer-implemented method comprising:

- obtaining (201) a training dataset (214) comprising workload data (212) and heart rate data (211) measured, by respectively at least one workload sensor and at least one heart rate sensor, during respective physical activities performed by the subject within a historical window;
- training (202) a recurrent neural network, RNN (220), based on the training dataset (214); wherein the RNN (220) is configured to determine the heart rate (221) of the subject at an exerted workload (222) considering a historic time series of heart rate data (223) and workload data preceding the exerted workload; and
- determining (203) at least a portion of a workload-heart rate relationship (263), indicative for the endurance performance of the subject, by feeding one or more fixed workload time series (241, 242, 243) to the trained RNN (220).

**2.** The computer-implemented method according to claim 1, wherein obtaining the training dataset comprises selecting (302) respective physical activities (314, 316, 317, 319, 320, 321) performed within the historical window (311) that meet a set of selection criteria (340).

**3.** The computer-implemented method according to any of the preceding claims, wherein obtaining the training dataset comprises oversampling (304) the workload data and the heart rate data of physical activities (316) that exceed a predetermined intensity.

**4.** The computer-implemented method according to any of the preceding claims, wherein obtaining the training dataset comprises selecting (303) respective initial portions (323) of the workload data and the heart rate data of the respective physical activities (316); wherein the initial portions have a predetermined length.

**5.** The computer-implemented method according to any of the preceding claims, wherein obtaining the training dataset comprises weighting (306, 330) the heart rate data values (331) within the training dataset inversely according to their frequency.

**6.** The computer-implemented method according to any of the preceding claims, wherein training the RNN further comprises finetuning (307) one or more layers of the RNN on a subset (341) of most recent physical activities within the training dataset.

**7.** The computer-implemented method according to any of the preceding claims, further comprising detecting deviations within a most recent physical activity (321) by excluding the most recent physical activity from the training dataset and comparing the heart rate data measured during the most recent physical activity (321) with the heart rate data determined by the RNN when feeding the workload data measured during the most recent physical activity to it.

8. The computer-implemented method according to any of the preceding claims, further comprising determining (401) the workload (412) of the subject at an aerobic threshold (421) based on the determined workload-heart rate relationship (243) and a predetermined heart rate of the subject at the aerobic threshold (411).

9. The computer-implemented method according to any of the preceding claims, further comprising determining (402) the workload (414) of the subject at an anaerobic threshold (422) based on the determined workload-heart rate relationship (243) and a predetermined heart rate of the subject at the anaerobic threshold (413).

10. The computer-implemented method according to any of claims 1 - 7, further comprising determining the workload and/or the heart rate of the subject at an aerobic threshold (421) and/or at an anaerobic threshold (422) by identifying (501) respective deflection points of the determined workload-heart rate relationship (243).

11. The computer-implemented method according to claim 9 or 10, further comprising determining (602) parameter values (614, 615, 616, 617) of a fitness-fatigue model (614) by minimizing (603) a difference between the workload at the anaerobic threshold (618) estimated by the fitness-fatigue model and the determined workload at the anaerobic threshold (414).

12. The computer-implemented method according to claim 11, further comprising determining (601) a training load (613) of at least one physical activity based on the workload data (610) and/or the heart rate data (611) of the at least one physical activity, wherein the training load is indicative for stress exerted on the subject by the at least one physical activity; and wherein the fitness-fatigue model (614) determines the performance of a subject based on the parameter values (614, 615, 616, 617) and the training load (613).

13. The computer-implemented method according to any of claims 11 - 12, wherein the determined parameter values of the fitness-fatigue model comprise:

- a first scaling factor (614) indicative for an increase in performance due to the fitness effect of the physical activity;
- a second scaling factor (615) indicative for a decrease in performance due to the fatigue effect of the physical activity;
- a first time factor (616) indicative for a decay of the impact of training load on the increase in performance over time; and
- a second time factor (617) indicative for a decay of the impact of training load on the decrease in performance over time.

14. The computer-implemented method according to claim 13, wherein determining (602) the parameter values (614, 615, 616, 617) of the fitness-fatigue model (614) further comprises constraining the values of the first scaling factor (614) and the second scaling factor (615) between at least 0.01 and at most 3.00, constraining the value of the first time factor (616) between at least 10 days and at most 60 days, and constraining the value of the second time factor (617) between at least 1 day and at most 10 days.

15. The computer-implemented method according to claim 13 or 14, wherein the second scaling factor (615) is at least equal to the first scaling factor (614); and a difference between the first time factor (616) and the second time factor (617) is at least 10 days.

110
111
LACTATE CONCENTRATION
114
$L_{LT2}$
113
$L_{LT1}$
118
119
117
$WL_{LT1}$
115
$WL_{LT2}$
116
WORKLOAD
112

Fig. 1

200
201
Obtaining a training dataset
$T = [(HR, WL)_{i-n}, \ldots, (HR, WL)_i]$
210
211
212
213
214
202
Training Recurrent Neural Network
$HR_{RNN}(t_n) = g(WL(t_n), HR_{RNN}(t_{n-1}), h(t_{n-1}))$
221
222
223
224
203
Determining at least a portion of a workload-heart rate relationship
240
WORKLOAD
241
242
243
$WL_1$
$WL_2$
$WL_3$
244
245
246
TIME
RNN
220
250
HEART RATE
251
252
253
254
255
256
TIME
260
261
HEART RATE
$HR_3$
$HR_2$
$HR_1$
254
255
256
263
264
265
266
$WL_1$
$WL_2$
$WL_3$
244
245
246
WORKLOAD
262

Fig. 2

300
SELECTION CRITERIA
340
Obtaining workload data and heart rate data measured during physical activities performed within historical window
301
Selecting physical activities
302
Selecting an initial portion of the physical activities
303
Oversampling high intensity physical activities
304
Standardizing WL and HR data of the initial portions
305
Weighting HR data values
306
Training Recurrent Neural Network
202
Finetuning one or more layers of the RNN on a subset of the training dataset
307
Determining at least a portion of a workload-heart rate relationship
203
311
310
312
313
314
315
316
317
318
319
320
321
316
322
323
324
330
331
332
counts
0.030
0.025
0.020
0.015
0.010
0.005
0.000
50
75
100
125
150
175
200
225
heart rate
340
341
314
316
317
319
320
321

Fig. 3

400
203
401
Determining $WL_{LT1} = f^{-1}(HR_{LT1})$
402
Determining $WL_{LT2} = f^{-1}(HR_{LT2})$
411
$HR_{LT1}$
413
$HR_{LT2}$
412
$WL_{LT1}$
414
$WL_{LT2}$
420
261
HEART RATE
262
WORKLOAD
263
421
422

Fig. 4

500
203
Identifying respective inflection points of the workload-heart rate relationship
501
HEART RATE
261
413
$HR_{LT2}$
411
$HR_{LT1}$
504
421
422
263
502
505
$WL_{LT1}$
412
$WL_{LT2}$
414
WORKLOAD
262

Fig. 5

600
600a
Determining $WL_{LT2}$
612
$WL_{LT2}$
601
Determining training load
613
$w_i$
602
Determining parameter values of a fitness-fatigue model
603
Minimizing $P_{LT2} - WL_{LT2}$
610
$WL\ DATA$
611
$HR\ DATA$
612
$WL_{LT2}$
614
$P_{LT2} = g(P_0, w_i, n, k_1, k_2, \tau_1, \tau_2)$
618
614
615
616
617

Fig. 6

740
700
704
702
716
710
706
708
714
712
720
730
751
752

Fig. 7

Europäisches Patentamt
European Patent Office
Office européen des brevets

# EUROPEAN SEARCH REPORT

Application Number
EP 24 20 1053

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | MELANIE LUDWIG ET AL: "A Convolution Model for Heart Rate Prediction in Physical Exercise :", PROCEEDINGS OF THE 4TH INTERNATIONAL CONGRESS ON SPORT SCIENCES RESEARCH AND TECHNOLOGY SUPPORT, 1 January 2016 (2016-01-01), pages 157-164, XP055396653, DOI: 10.5220/0006030901570164 ISBN: 978-989-7582-05-9<br>* Abstract, 2 HEART RATE PREDICTION DURING EXERCISE, 3 THE CONVOLUTION MODEL APPROACH, 4 EXPERIMENTS *<br>----- | 1,2,4-7<br>3,8-15 | INV.<br>G16H20/30<br>G16H50/30<br>G06N3/08<br>G06N20/00 |
| A | US 2015/327804 A1 (LEFEVER JORIS [BE] ET AL) 19 November 2015 (2015-11-19)<br>* paragraph [0018] *<br>----- | 1-15 | |
| A | MAZZOLENI MICHAEL J ET AL: "Modeling and predicting heart rate dynamics across a broad range of transient exercise intensities during cycling", SPORTS ENGINEERING, SPRINGER LONDON, LONDON, vol. 19, no. 2, 19 January 2016 (2016-01-19), pages 117-127, XP035691828, ISSN: 1369-7072, DOI: 10.1007/S12283-015-0193-3 [retrieved on 2016-01-19]<br>* abstract *<br>----- | 1-15 | TECHNICAL FIELDS SEARCHED (IPC)<br>G16H<br>G06N |

The present search report has been drawn up for all claims

| Place of search | Date of completion of the search | Examiner |
|---|---|---|
| Munich | 25 February 2025 | Reinbold, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 20 1053

This annex lists the patent family members relating to the patent documents cited in the above-mentioned European search report. The members are as contained in the European Patent Office EDP file on The European Patent Office is in no way liable for these particulars which are merely given for the purpose of information.

25-02-2025

| Patent document cited in search report | Publication date | Patent family member(s) | Publication date |
|---|---|---|---|
| US 2015327804 A1 | 19-11-2015 | AU 2013220945 A1 | 02-10-2014 |
| | | EP 2815344 A1 | 24-12-2014 |
| | | US 2015327804 A1 | 19-11-2015 |
| | | WO 2013120151 A1 | 22-08-2013 |

EPO FORM P0459

For more details about this annex : see Official Journal of the European Patent Office, No. 12/82